# EUROPEAN PATENT SPECIFICATION

(11) **EP 3 334 290 B1**
(45) Date of publication and mention of the grant of the patent: **30.12.2020**
(21) Application number: 16758279.0
(22) Date of filing: 05.08.2016
(51) Int. Cl.: A23K 50/40, A23L 33/00, A61P 3/04

(54) **METHODS USING MORPHOMETRIC MEASUREMENTS OF A SMALL DOG TO IMPROVE FOOD FOR THE SMALL DOG**
METHODEN WOBEI MORPHOMETRISCHE MESSUNGEN EINES KLEINEN HUNDES SIND VERWENDET, UM DESSEN ERNÄHRUNG ZU VERBESSERN
METHODES UTILISANT DES MESURES MORPHOMETRIQUES D'UN CHIEN DE PETITE TAILLE POUR AMELIORER SON ALIMENTATION

(30) Priority: 11.08.2015 US 201562203710 P; 15.10.2015 US 201562242107 P
(43) Date of publication of application: 20.06.2018
(73) Proprietor: Société des Produits Nestlé S.A., 1800 Vevey (CH)
(72) Inventor: BOUTHEGOURD, Jean-Christophe, 95110 Sannois (FR); GORE, Asa, St. Louis, Missouri 63129 (US)
(74) Representative: Elkington and Fife LLP
(86) International application number: PCT/IB2016/054748
(87) International publication number: WO 2017/025881

(56) References cited:
- WO-A2-2007/079386
- US-A1- 2010 233 323
- US-A1- 2014 272 028
- US-B2- 6 691 639
- FINKE M D: "EVALUATION OF THE ENERGY REQUIREMENTS OF ADULT KENNEL DOGS", THE JOURNAL OF NUTRITION, AMERICAN SOCIETY FOR NUTRITION, US, vol. 121, no. 11, SUPPL, 1 January 1991 (1991-01-01), pages S22-S28, XP001024531, ISSN: 0022-3166
- KIENZLE E ET AL: "MAINTENANCE ENERGY REQUIREMENT OF DOGS: WHAT IS THE CORRECT VALUE OF CALCULATION OF METABOLIC BODY WEIGHT IN DOGS?", THE JOURNAL OF NUTRITION, AMERICAN SOCIETY FOR NUTRITION, US, vol. 121, no. SUPPL. 11, 1 January 1991 (1991-01-01), page S39/S40, XP008066982, ISSN: 0022-3166
- Wsava ET AL: "Body Condition Score", , 1 December 2013 (2013-12-01), XP055312249, Retrieved from the Internet: URL:http://www.wsava.org/sites/default/fil es/Body condition score chart dogs.pdf [retrieved on 2016-10-19]

## Description

### BACKGROUND

The present disclosure relates generally to methods for preparing dog food compositions. More specifically, the present disclosure is directed to methods of preparing dog food compositions that are formulated based on morphometric measurements of a small dog that will consume the food composition.

Foods adapted to a canine's age or size can be found on the market. For example, canine foods intended for puppies, adults, and senior canines are well known and available for purchase at most retail outlets that sell dog foods. Similarly, canine foods intended for overweight canines or canines with particular conditions or diseases are known.

U.S. Patent No. 6,156,355 discloses foods that are designed for specific canine breeds. However, variables other than age, size, and heath distinguish canines of different breeds and affect their food requirements. Food based solely on breed, size or utility do not adequately achieve the appropriate nutritional needs of a dog.

For example, the influence of breed, body weight, age, and gender on energy requirements has been investigated. The results show that some animals having the same body weight have very different energy requirements. Other studies have shown that age-related changes are observed in the metabolism and body composition of Papillons, Labrador Retrievers, and Great Danes and that these changes affect life expectancy. Studies have shown that some differences exist in terms of body composition and resting metabolic rate and that these differences are independent of body weight. For example, feeding care will differ between a 30 kilogram (kg) Labrador and a 30 kg Greyhound of the same age and gender and living in the same environmental conditions because their genetic backgrounds are different and these differences result in different metabolisms and body compositions. For example, the Greyhound might require more energy per kg of ideal body weight (Kcal per day=150xKg Body Weight^{0.75}) to stay in ideal body condition, and the Labrador might require less energy (Kcal per day=110xKg Body Weight^{0.75}). Methods of preparing pet foods taking into account the condition or conformation of the body are disclosed in e.g., US 2010/0233323 A1, US 6691639 B2, and US 2014/0272028 A1.

The selection of a particular food for an individual dog or class of dog is often confusing. The consumer must determine the class and type of the dog and select from foods, none of which may be designed for the particular animal that will consume the food.

### SUMMARY

The present inventors surprisingly found a new parameter for classifying small dogs into one of a plurality of body categories, preferably a robust physique category or an athletic physique category. For example, the inventors considered defining body mass index ("BMI") as body weight / (height of the shoulder x body length) (kg/m²). However, the present inventors found this calculation may not be accurate enough in small dogs because of very small legs in some breeds like dachshund that resulted in wrong classification. The present inventors found that a calculation of BMI as body weight / [(height - height of the sternum to the floor) x body length] was more effective in classifying dogs, particularly small breeds, than the standard BMI calculation. In some embodiments, the categorizing of the small dog can also use skin fold measurements.

Accordingly, in a general embodiment, the present disclosure provides a method of formulating a dog food composition for a small dog having a height, a sternum and a body length, the method comprising: generating, while the small dog is erect on a floor, measurements of the height, the height of the sternum to the floor, and the body length; using the measurements to determine the modified body mass index of the small dog, wherein said modified body mass index is body weight / [(height - height of the sternum to the floor) x body length]; categorizing the small dog as one of a plurality of body conformation types, the categorizing being based at least partially on a modified body mass index of the small dog; and formulating a dog food composition for the small dog, wherein the dog food composition is formulated to contain feed ingredients and amounts thereof based on the categorizing of the small dog.

In an embodiment, the categorizing of the small dog categorizes the small dog as a robust body conformation type or an athletic body conformation type, the modified body mass index of the robust body conformation type is greater than 60 kg/m², and the modified body mass index of the athletic body conformation type is not greater than 60 kg/m².

In an embodiment, a ratio of energy from protein to energy from fat is different for the dog food composition formulated for the small dog categorized as the robust body conformation type relative to the dog food composition formulated for the small dog categorized as the athletic body conformation type. The ratio of energy from protein to energy from fat can be greater for the dog food composition formulated for the small dog categorized as the robust body conformation type relative to the dog food composition formulated for the small dog categorized as the athletic body conformation type.

In an embodiment, a fat content is different for the dog food composition formulated for the small dog categorized as the robust body conformation type relative to the dog food composition formulated for the small dog categorized as the athletic body conformation type. The fat content can be greater for the dog food composition formulated for the small dog categorized as the athletic body conformation type relative to the dog food composition formulated for the small dog categorized as the robust body conformation type.

In an embodiment, the small dog has a shoulder, a sternum and a body length; the method comprises generating, while the small dog is erect on a floor, measurements of the height of the shoulder to the floor, the height of the sternum to the floor, and the body length; and the categorizing of the small dog comprises using the measurements to determine the modified body mass index.

In an embodiment, the categorizing is partially based on an energy requirement of the small dog. The categorizing of the small dog can categorize the small dog as a robust body conformation type or an athletic body conformation type; for the robust body conformation type, the modified body mass index is greater than 60 kg/m² and/or the energy requirement is less than 120x(kilograms of ideal body weight)^{0.75} kilocalories per day; and for the athletic body conformation type, the modified body mass index is not greater than 60 kg/ m² and/or the energy requirement is more than 120x(kilograms of ideal body weight)^{0.75} kilocalories per day.

In an embodiment, the categorizing is partially based on a skin fold measurement of the small dog, the skin fold measurement is one or more measurements of the thickness of the subcutaneous fat layer of the small dog, and each of the one or more measurements is obtained in a different location on the small dog relative to the other measurements.

In an embodiment, the small dog has a weight between 5 and 10 kg.

In an embodiment, the small dog has a weight less than 5 kg.

In another embodiment, the present invention provides a method of selecting a food composition for a small dog having a height, a sternum, and a body length. The method comprises: measuring, while the small dog is erect on a floor, the height, the height of the sternum to the floor, and the body length; determining a modified body mass index of the small dog, the modified body mass index is body weight / [(height - height of the sternum to the floor) x body length]; categorizing the small dog as one of a plurality of body conformation types using the modified body mass index; and selecting one of a plurality of food compositions based on the categorizing, each of the plurality of food compositions respectively corresponds to one of the plurality of body conformation types.

In an embodiment, a first food composition of the plurality of food compositions is formulated for a robust body conformation type of the small dog, the modified body mass index of the robust body conformation type is greater than 60 kg/m², a second food composition of the plurality of food compositions is formulated for an athletic body conformation type of the small dog, and the modified body mass index of the athletic body conformation type is not greater than 60 kg/m².

In an embodiment, the first food composition comprises a functional ingredient which limits fat accumulation selected from the group consisting of conjugated linoleic acid (CLA), isoflavones, and combinations thereof, and the second food composition does not contain the functional ingredient which limits fat accumulation.

In an embodiment, a ratio of energy from protein to energy from fat is different for the first food composition relative to the second food composition.

In an embodiment, a fat content is different for the first food composition relative to the second food composition.

In an embodiment, the categorizing is partially based on a skin fold measurement of the small dog, the skin fold measurement is one or more measurements of the thickness of the subcutaneous fat layer of the small dog, and each of the one or more measurements is obtained in a different location on the small dog relative to the other measurements.

In another embodiment, the method provides a dog food composition, said method comprising: categorizing a small dog as one of a plurality of body conformation types, the categorizing based at least partially on a modified body mass index of the small dog, the modified body mass index is body weight / [(height - height of the sternum to the floor) x body length]; and formulating the dog food composition for the small dog, the dog food composition is formulated to contain feed ingredients and amounts thereof based on the categorizing of the small dog.

An advantage of one or more embodiments provided by the present disclosure is to prepare dog food compositions formulated for small dogs having certain morphometric measurements.

Another advantage of one or more embodiments provided by the present disclosure is prepare dog food compositions formulated for robust and athletic dogs that are small dogs.

A further advantage of one or more embodiments provided by the present disclosure is to better classify small breeds relative to known methods.

Yet another advantage of one or more embodiments provided by the present disclosure is to consider leg length when calculating BMI of a small dog.

Another advantage of one or more embodiments provided by the present disclosure is to provide a better BMI calculation for small canines relative to known BMI calculations.

A further advantage of one or more embodiments provided by the present disclosure is to improve healthy weight management of small canines relative to known products and diets for such canines.

Still another advantage of one or more embodiments provided by the present disclosure is to overcome difficulties in differentiating small breeds with a same body weight.

Another advantage of one or more embodiments provided by the present disclosure is to provide simplified methods to prepare and select dog food compositions for a particular small dog or class of small dog.

A further advantage of one or more embodiments provided by the present disclosure is to use several variables to categorize a small dog based upon body conformation type and prepare and/or select an appropriate food composition for the dog.

Still another advantage of one or more embodiments provided by the present disclosure is to achieve food compositions better adapted for the small dog than traditional food compositions.

Yet another advantage of one or more embodiments provided by the present disclosure is to enable a consumer to simply select an appropriate food composition based upon the dog body conformation type of the consumer's small dog.

Additional features and advantages are described herein and will be apparent from the following Detailed Description and the Figures.

### BRIEF DESCRIPTION OF THE FIGURES

**FIG. 1** shows the morphometric measurements obtained in the study detailed herein.
**FIGS. 2-6** are charts showing the data from the study detailed herein.
**FIG. 7** is a graph of BMI and skin folds as parameters to classify robust and athletic small dogs based on the data from the study detailed herein.
**FIG. 8** is a graph of PCA mapping of small dog classes from the data in the study detailed herein.

### DETAILED DESCRIPTION

As used in this disclosure and the appended claims, the singular forms "a," "an" and "the" include plural referents unless the context clearly dictates otherwise. Thus, for example, reference to "a dog" or "the dog" includes two or more dogs.

The words "comprise," "comprises" and "comprising" are to be interpreted inclusively rather than exclusively. Likewise, the terms "include," "including" and "or" should all be construed to be inclusive, unless such a construction is clearly prohibited from the context.

However, the compositions disclosed herein may lack any element that is not specifically disclosed. Thus, a disclosure of an embodiment using the term "comprising" includes a disclosure of embodiments "consisting essentially of" and "consisting of" the components identified. Similarly, the methods disclosed herein may lack any step that is not specifically disclosed herein. Thus, a disclosure of an embodiment using the term "comprising" includes a disclosure of embodiments "consisting essentially of" and "consisting of" the steps identified.

The term "and/or" used in the context of "X and/or Y" should be interpreted as "X," or "Y," or "X and Y." Where used herein, the terms "example" and "such as," particularly when followed by a listing of terms, are merely exemplary and illustrative and should not be deemed to be exclusive or comprehensive. Any embodiment disclosed herein can be combined with any other embodiment disclosed herein unless explicitly stated otherwise.

Numerical adjectives, such as "first" and "second," are merely used to distinguish components. These numerical adjectives do not imply the presence of other components, a relative positioning, or any chronological implementation. In this regard, the presence of a "second food composition" does not imply that a "first food composition" is necessarily present. Further in this regard, a "second food composition" can be used before, after, or simultaneously with any "first food composition."

All percentages expressed herein are by weight of the total weight of the composition unless expressed otherwise. As used herein, "about" and "approximately" are understood to refer to numbers in a range of numerals, for example the range of -10% to +10% of the referenced number, preferably within -5% to +5% of the referenced number, more preferably within -1% to +1% of the referenced number, most preferably within -0.1% to +0.1% of the referenced number. All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

The term "dog" means all canine animals, non-limiting examples of which include pet dogs, working dogs, show dogs, guard dogs, scent hounds, and retrievers. The term "puppy" means an animal of any age in the juvenile growth and development stage. For example, a dog that is a "puppy" typically has an age up to about 1 year. The term "adult" means an animal of an age after the completion of the juvenile growth and development stage until development of an increased risk of age-related disease. For example, a dog that is an "adult" typically has an age from about 1 year to about 7 years. The term "senior" means an animal of an age having an increased risk for age-related disease but may or may not have obvious physical or behavioral characteristics of aging. For example, a dog that is a "senior" typically has an age from about 7 years to about 11 years. The term "geriatric" means an animal showing outward signs of aging. For example, a dog that is "geriatric" typically has an age of about 11 years or more.

The term "small dog" means a dog that weighs less than 10 kg. The term "miniature dog" means a dog that weighs less than 5 kg, and miniature dogs are encompassed by the term "small dog." The term "medium dog" means a dog that weighs between 10 and 25 kg. The term "large dog" means a dog that weighs between 25 and 40 kg. The term "giant dog" means a dog that weighs more than 40 kg.

The term "food composition" means any composition suitable for consumption by a dog, including, but not limited to, dry, wet, semi-moist, moist, and liquid food compositions.

The term "kit" encompasses single packages and virtual packages. A "single package" means that the components of the kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual devices and/or food compositions physically associated with each other such that they are considered a unit for manufacture, distribution, sale, or use. A "virtual package" means that the components of the kit are associated by directions on one or more physical or virtual kit components instructing the user how to obtain the other components, e.g., a bag containing one component and directions instructing the user to go to a website, contact a recorded message, view a visual message, or contact a caregiver or instructor to obtain instructions on how to use the kit.

The methods and devices and other advances disclosed herein are not limited to particular methodologies, protocols, and reagents because, as the skilled artisan will appreciate, they may vary. Further, the terminology used herein is for the purpose of describing particular embodiments only and does not limit the scope of that which is disclosed or claimed.

Unless defined otherwise, all technical and scientific terms, terms of art, and acronyms used herein have the meanings commonly understood by one of ordinary skill in the art in the field(s) of the present disclosure or in the field(s) where the term is used. Although any compositions, methods, articles of manufacture, or other means or materials similar or equivalent to those described herein can be used, the preferred devices, methods, articles of manufacture, or other means or materials are described herein.

The invention is defined by the claims. An aspect of the present disclosure is methods for preparing dog food compositions adapted for small dogs with certain body conformation types. The methods can comprise (1) categorizing a small dog for which a food composition is intended, wherein the small dog is categorized as a robust or athletic body type conformation, and (2) formulating a dog food composition to comprise ingredients and amounts thereof that are beneficial for the categorized small dog.

As noted above, the present inventors surprisingly found that a calculation of BMI as body weight / [(height - height of the sternum to the floor) x body length] was more effective in classifying small dogs than the standard BMI calculation. The improved BMI calculation is referenced hereafter as "BMI 3." In a preferred embodiment, the small dog can be categorized as a robust dog or an athletic dog.

In an embodiment, a robust dog is a small dog that has a BMI 3 greater than 60 kg/m². The small dog can also be categorized based on an energy requirement of the small dog. In such an embodiment, a robust dog is a small dog that has a BMI 3 greater than 60 kg/m² and an energy requirement of less than 120x(kilograms of ideal body weight)^{0.15} kilocalories per day. The robust dog can have at least one additional characteristic selected from the group consisting of (1) a thorax/waist circumference ratio less than 1.5; (2) a low body surface/kg; (3) a fat to muscle ratio greater than 1; and (4) a fat mass greater than 15% (e.g., a fat mass of 15-20% of the total body mass).

In an embodiment, an athletic dog is a small dog that has a BMI 3 not greater than 60 kg/m². The small dog can also be categorized based on an energy requirement of the small dog. In such an embodiment, an athletic dog is a small dog that has a BMI 3 not greater than 60 kg/m² and an energy requirement of more than 120X(kilograms of ideal body weight)^{0.75} kilocalories per day. The athletic dog can have at least one additional characteristic selected from the group consisting of (1) a thorax/waist circumference ratio greater than 1.5; (2) a high body surface/kg; (3) a fat to muscle ratio less than 1; and (4) a fat mass less than 15% (e.g., fat a mass of 10-15% of the total body mass).

The dog may be further classified as a puppy, an adult dog, a senior dog, or a geriatric dog. The dog may be classified as miniature, small, medium, large or giant based upon their weight.

As noted above, a small dog is classified as either robust or athletic based upon the dog's body conformation type. Generally, body type conformations are influenced by and dependent upon a variety of factors, including the body mass index, body composition, daily energy requirement, resting metabolic rate, dog breed, and genetics differentiation during breeding history. Given the body conformation and body composition of robust and athletic dogs, athletic dogs may be metabolically more active than robust dogs. Therefore, to maintain ideal body condition in all dogs, robust dogs typically require less than 120x(kilograms of ideal body weight)^{0.75} kilocalories per day. In contrast, athletic dogs typically require more than 120x(kilograms of ideal body weight)^{0.75} kilocalories per day.

The resting metabolic rate is a measure of the energy utilized when a dog is in a resting state as a function of time. An RMR (resting metabolic rate) greater than 10,000 kJ/day can characterize an athletic dog, whereas an RMR of less than 10000 kJ/day can characterize a robust dog. Athletic dogs generally have a RMR which is about 57% higher than the RMR of robust dogs.

Categorizing a dog as robust or athletic may also be influenced by the breeding history of a dog. For instance, dogs may have a different breeding history and genetic background than the breed category in which they are categorized. Generally, dogs having some athletic blood in their breeding history tend to have kept the athletic morphology as a dominant phenotype and have higher energy needs. For example, the Great Dane that belongs to the working and guard dog group (and therefore should be classified as a robust dog) may be classified as athletic because of its morphology and breeding history (sight hounds blood). It has a clear athletic type body conformation, i.e., deep chest and thin abdomen and high daily energy requirements to maintain his ideal body weight.

The second step in the methods disclosed herein is formulating a dog food composition, for example the protein and fat content thereof, based on whether a small dog is classified as robust or as athletic.

The dog food composition for a robust small dog preferably comprises comestible ingredients that provide a ratio of energy from protein to energy from fat greater than 0.80. Preferably, the food composition for a robust small dog has a protein content of about 20% to about 30% on a dry matter basis and a fat content less than about 15% on a dry matter basis.

The dog food composition for an athletic dog preferably comprises comestible ingredients that provide a ratio of energy from protein to energy from fat less than 0.80. Preferably, the food composition has a protein content of from about 20-30% on a dry matter basis and a fat content of about 15% to about 25% on a dry matter basis.

Activity level and type differ according to body type conformation. For example, a robust small dog will be spontaneously engaged in mild (e.g., slow walking), moderate (e.g., brisk walking) or occasionally intense (e.g., running) activity types. An athletic small dog, in comparison, will mainly be voluntary involved in moderate, intense or very intense (e.g., fast running) activities. Within these different levels of activity, dogs can be further classified as robust or athletic.

A dog food composition having a ratio of energy from protein to energy from fat below 0.80 has been found to be advantageous to athletic small dogs. A food composition high in protein and high in fat is particularly well adapted for athletic small dogs. Typically, a dog food composition for athletic dogs has about 20% to about 30% protein and about 15% to about 25% fat. A food composition dense in energy from fat can provide an athletic small dog with sufficient energy for the moderate to very intense activities (i.e., brisk walk to fast run) in which the dog is spontaneously involved. Furthermore, the ratio of the energy from protein to the energy from fat can be advantageous in such a food composition for maintaining the lean body mass of athletic small dogs.

A food composition for a robust small dog can have a ratio of energy from protein to energy from fat greater than 0.80. Typically, a dog food composition for robust small dogs has about 20% to about 30% protein and less than about 15% fat. Robust small dogs typically have a low resting metabolic rate, thus such a food composition can be ideally adapted to robust small dogs. The composition can have the effect of limiting the fat intake of robust small dogs and therefore their tendency to be overweight.

Functional ingredients may also be used in the food compositions for athletic small dogs and/or the food compositions for robust small dogs. Functional ingredients mean any ingredient which confers a health benefit to the dog consuming the food composition to which the ingredient is added. Non-limiting examples of suitable functional ingredients include carbohydrates, fibers, vitamins, minerals, prebiotics, probiotics, and combinations thereof. The food composition for the robust small dog can include functional ingredients which limit fat accumulation, non-limiting examples of which include conjugated linoleic acid (CLA), isoflavones, and combinations thereof.

When the small dog is fed a food composition according to the present disclosure, the feeding recommendations can be adjusted depending on whether the dog is robust or athletic. Depending on their level of activity (i.e., mild, moderate or intense) and frequency, the robust small dogs can require an energy intake (in kilocalories) less than 120 x (kilograms of ideal body weight)^{0.75} kilocalories per day. Depending on their level of activity (i.e., moderate, intense or very intense), the athletic small dogs can require an energy intake (in kilocalories) more than 120 x (kilograms of ideal body weight)^{0.75} kilocalories per day.

Typical dog food ingredients used in the preparation of a dog food comprise meat sources (e.g., chicken, lamb etc.), other protein sources (e.g., dehydrated poultry protein, dried whole egg, etc.), rice, maize, wheat, animal fat, fiber (e.g., beet pulp, oat fiber etc.), oil (e.g., sunflower oil, fish oil etc.), yeast, amino acids, minerals and functional ingredients. The step of formulating the dog food composition based on the classification of the small dog can comprise selecting specific ingredients and/or specific amounts thereof based on the classification, e.g. robust or athletic.

For example, the food composition can contain one or more of meats, grains, vegetable proteins, fiber, vitamins, minerals and fats. Non-limiting examples of meats suitable for the compositions disclosed herein include poultry, beef, pork, lamb and fish, especially those types of meats suitable for pets. Some or all of the meat can be provided as one or more meat meals, namely meat that has been dried and ground to form substantially uniform-sized particles and as defined by the Association of American Feed Control Officials (AAFCO). The step of formulating the dog food composition based on the classification of the small dog can comprise selecting a specific meat and/or a specific amount thereof based on the classification, e.g. robust or athletic.

Non-limiting examples of suitable grains include corn, rice, wheat, barley, oats, soy, sorghum, millet, triticale, rye and mixtures thereof, preferably in whole grain form. Non-limiting examples of suitable vegetable proteins include wheat protein (e.g., whole grain wheat or wheat gluten such as vital wheat gluten), corn protein (e.g., ground corn or corn gluten), soy protein (e.g., soybean meal, soy concentrate, or soy isolate), rice protein (e.g., ground rice or rice gluten), cottonseed, peanut meal, pea protein, and combinations thereof. Some materials are both a vegetable protein and a grain. The step of formulating the dog food composition based on the classification of the small dog can comprise selecting a specific grain, a specific vegetable protein and/or a specific amount thereof based on the classification, e.g. robust or athletic.

Soluble fibers and/or insoluble fibers may be utilized. Non-limiting examples of suitable fiber sources include chicory, cellulose, beet pulp (from sugar beet), gum arabic, gum talha, psyllium, rice bran, carob bean gum, citrus pulp, pectin, fructooligosaccharide, short chain oligofructose, mannanoligofructose, soy fiber, arabinogalactan, galactooligosaccharide, arabinoxylan, and mixtures thereof. In an embodiment, an amount of fiber is used so that the final food composition comprises 1-10% of the fiber. The step of formulating the dog food composition based on the classification of the small dog can comprise selecting a specific fiber and/or a specific amount thereof based on the classification, e.g. robust or athletic.

The fiber source can be a fermentable fiber. Fermentable fiber has previously been described to provide a benefit to the immune system of a companion animal. Fermentable fiber or other compositions known to skilled artisans that provide a prebiotic to enhance the growth of probiotics within the intestine may be incorporated into the dry pet food.

Non-limiting examples of suitable fats include animal fats and vegetable fats. Preferably the fat source is an animal fat source, such as tallow or grease. Vegetable oils, such as corn oil, sunflower oil, safflower oil, rape seed oil, soy bean oil, olive oil and other oils rich in monounsaturated and polyunsaturated fatty acids, can be used additionally or alternatively. In some embodiments, a source of omega-3 fatty acids is included, such as one or more of fish oil, krill oil, flaxseed oil, walnut oil, or algal oil. The step of formulating the dog food composition based on the classification of the small dog can comprise selecting a specific fat and/or a specific amount thereof based on the classification, e.g. robust or athletic.

Non-limiting examples of suitable vitamins include vitamin A, any of the B vitamins, vitamin C, vitamin D, vitamin E, and vitamin K, including various salts, esters, or other derivatives of the foregoing. Non-limiting examples of suitable minerals include calcium, phosphorous, potassium, sodium, iron, chloride, boron, copper, zinc, magnesium, manganese, iodine, selenium, and the like. The step of formulating the dog food composition based on the classification of the small dog can comprise selecting a specific vitamin, a specific mineral and/or a specific amount thereof based on the classification, e.g. robust or athletic.

The food composition can include other additional components such as one or more of a preservative, a colorant or a palatant. Non-limiting examples of suitable preservatives include potassium sorbate, sorbic acid, sodium methyl para-hydroxybenzoate, calcium propionate, propionic acid, and combinations thereof. Non-limiting examples of suitable colorants include FD&C colors, such as blue no. 1, blue no. 2, green no. 3, red no. 3, red no. 40, yellow no. 5, yellow no. 6, and the like; natural colors, such as roasted malt flour, caramel coloring, annatto, chlorophyllin, cochineal, betanin, turmeric, saffron, paprika, lycopene, elderberry juice, pandan, butterfly pea and the like; titanium dioxide; and any suitable food colorant known to the skilled artisan. Non-limiting examples of suitable palatants include yeast, tallow, rendered animal meals (e.g., poultry, beef, lamb, and pork), flavor extracts or blends (e.g., grilled beef), animal digests, and the like.

In another aspect, the present disclosure provides a dog food composition prepared using any of the methods disclosed herein.

In yet another aspect, the present disclosure provides a package comprising a material suitable for containing a dog food composition adapted for consumption by a robust or an athletic dog, preferably a dog food composition prepared using any of the methods disclosed herein, and a label affixed to the package containing a word or words, picture, design, acronym, slogan, phrase, or other device, or combination thereof, that indicates that the contents of the package contains a food composition adapted for consumption by a robust or an athletic small dog. Typically, such device comprises the words "formulated for robust small dogs" or "formulated for athletic small dogs" or an equivalent expression printed on the package. Any package or packaging material suitable for containing dog food can be used, e.g., a bag, box, bottle, can, pouch, and the like manufactured from paper, plastic, foil, metal, and the like. In a preferred embodiment, the package contains a dog food composition adapted for a robust dog or an athletic dog, as appropriate for the label, preferably a dog food composition prepared using any of the methods disclosed herein.

### EXAMPLE

The following non-limiting example details a study involving small dogs to differentiate their athletic and robust physiques.

### Materials and Methods

Ninety-five entire or neutered dogs of two and a half year on average (45 males, 50 females) from miniature, small and medium classification of sixteen different breeds (Chihuahua; Dachshund wire-haired; Dachshund long-haired; Fox-terrier; Havanese; Italian Greyhound; Japanese Chin; Lhasa Apsso; Miniature Pinscher; Parson Russel Terrier; Pomeranian; Cairn Terrier; Cavalier King Charles; French Bulldog; Pug; West-highland White Terrier) were used in this study. Dogs were coming either from the animal petcare facility of Nestlé-Purina in Amiens, France (N=21) or were private particular-owned dogs (N=74). All the dogs were healthy based on physical examination.

### Morphometric measurements

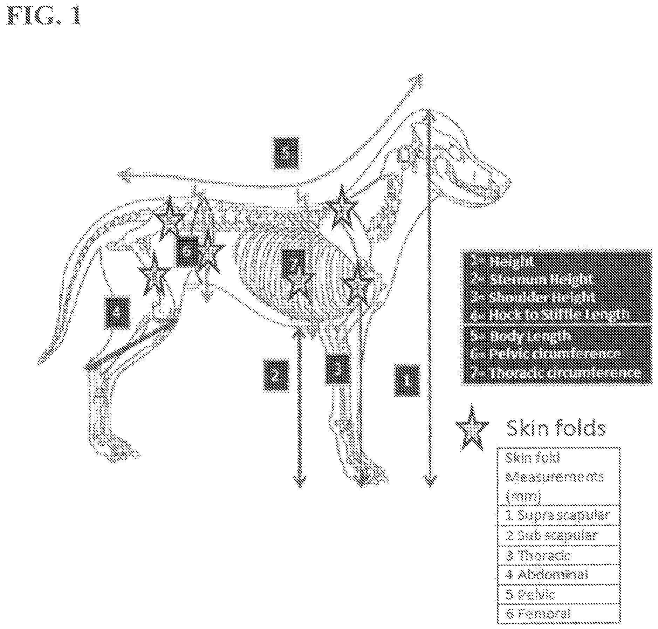

Body condition score (BCS) was performed on each dog using the 9 points scale validated by Laflamme et al., "Development and validation of a body condition score system for dogs," Canine Pract. 22:10-15 (1997). The body weight was taken with a scale of 100 g of precision. As shown in FIG. 1, the following morphometric measurements were performed on erect dogs with a cloth tape to the nearest centimeter: height, height sternum to floor, shoulder height, body length, pelvic circumference, and thoracic circumference.

The height was defined as the distance from the floor to the top of the head.

The sternum height (or height sternum to floor) was defined as the distance from the floor to the lowest point of the sternum.

The shoulder height was defined as the distance from the floor to the tip of the acromion.

Body length was the distance between the occiput and the base of the tail.

Thoracic circumference was measured by wrapping the tape around the dog where thorax is the widest.

Pelvic circumference was measured by wrapping the tape around the dog where pelvis is the thinnest.

The body weight was recorded in kilograms and the other measurements were recorded in centimeters. Based on these morphometric measurements, ratio between thoracic and pelvic circumferences and body mass indexes 1, 2 and 3 (BMI 1, BMI2 and BMI3) were calculated with the following equations: $BMI 1 = \frac{Body weight \left(kg\right)}{{\left(shoulder height \left(cm\right)\right)}^{2}}$ $BMI 2 = \frac{Body weight \left(kg\right)}{\left(shoulder height \left(cm\right)∗body length\left(cm\right)\right)}$ $BMI 3 = \frac{Body weight \left(kg\right)}{\left(height \left(cm\right)-height sternum to the floor \left(cm\right)∗body length\left(cm\right)\right)}$

### Skinfold measurement

A skinfold caliper was used on 36 different dogs to measure the thickness of the subcutaneous fat layer on six different zones assuming that the sum is representative of the total body fat. Measures were taken on supra-scapular, sub scapular, thoracic, abdominal, pelvic, femoral zones and recorded in centimeters. The sum of skinfold was calculated (Total Skinfold).

### Allometric factor

59 dogs were fed an adult diet (crude protein 26%, crude fat 16%, crude fiber 1.5%, 3700 kcal ME/kg, as is) and ration was calculated for maintenance based on the knowledge of dog's needs. Daily food intake was recorded to calculate allometric factor for each dog defined with the following equation: $Allometric factor = \frac{Food intake for weight maintenance \left(kg\right)}{{\left(Body weight\right)}^{0.75}}$

### Data Analysis

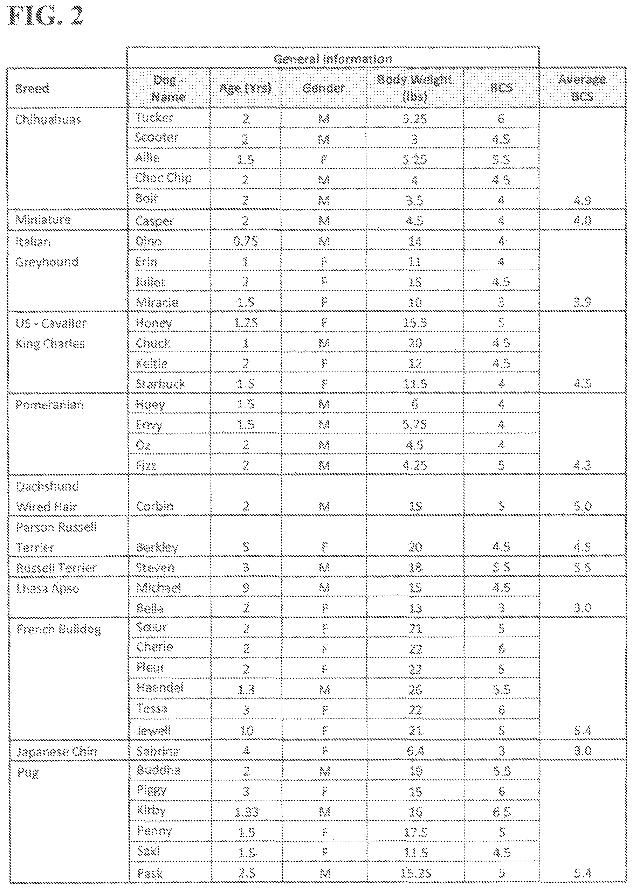

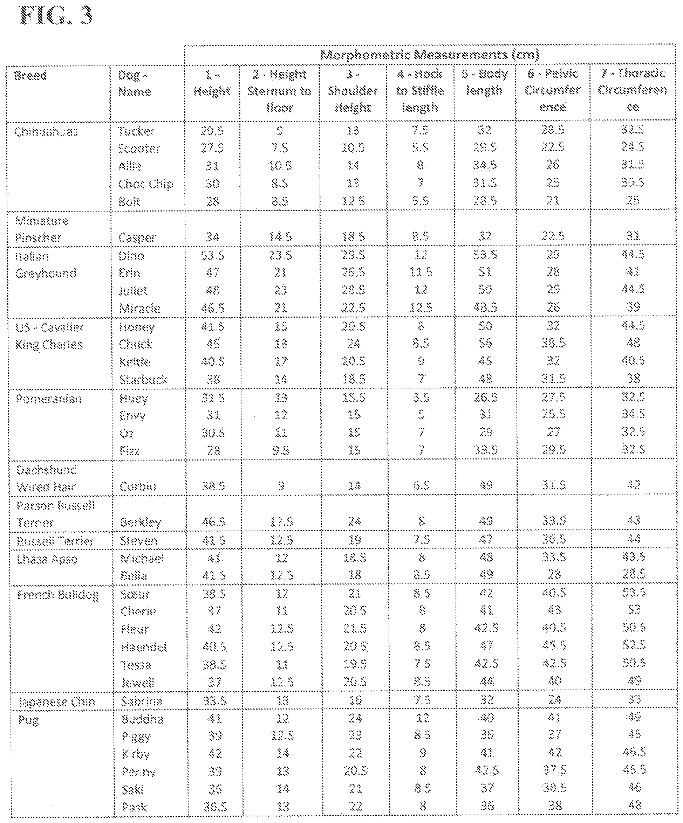

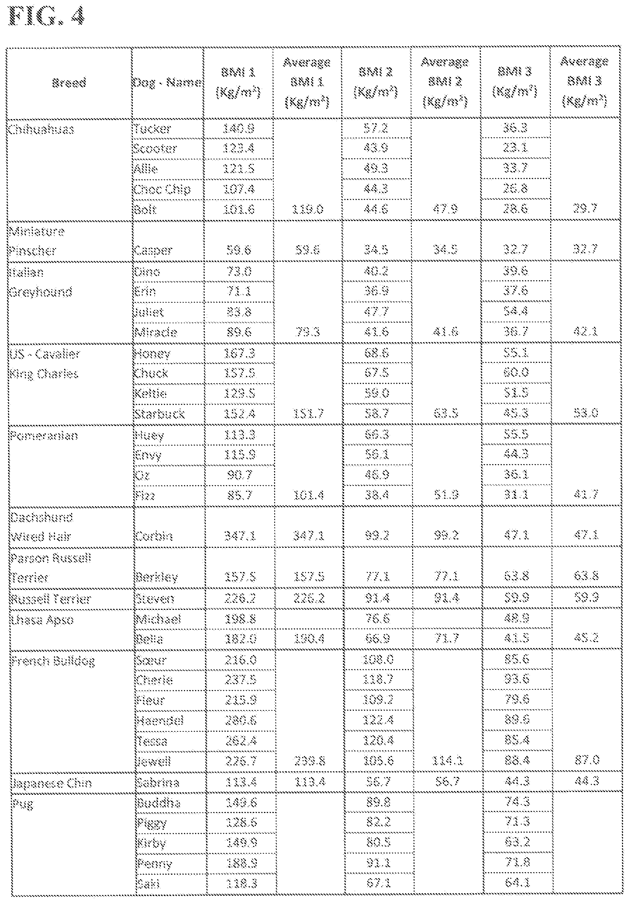

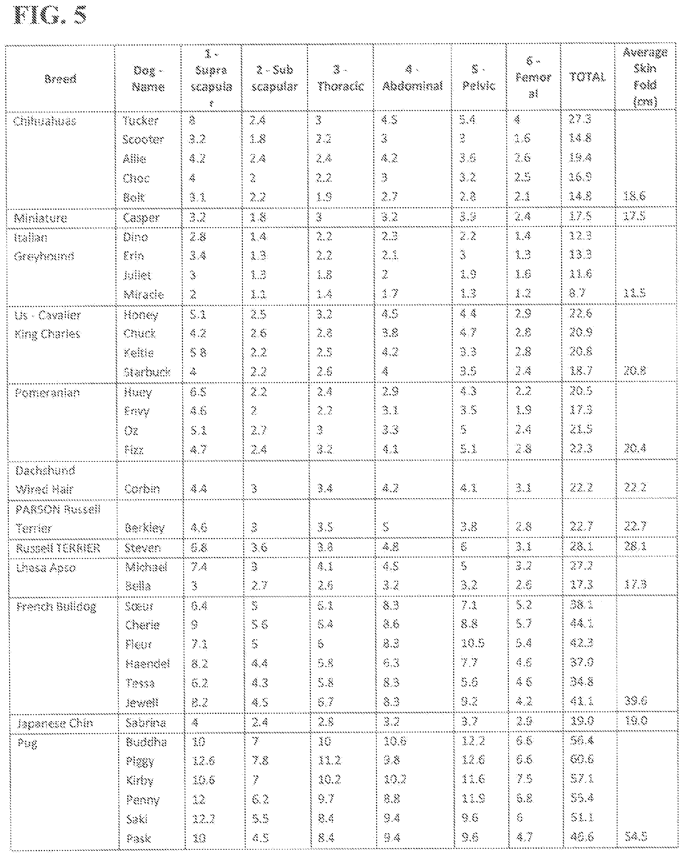

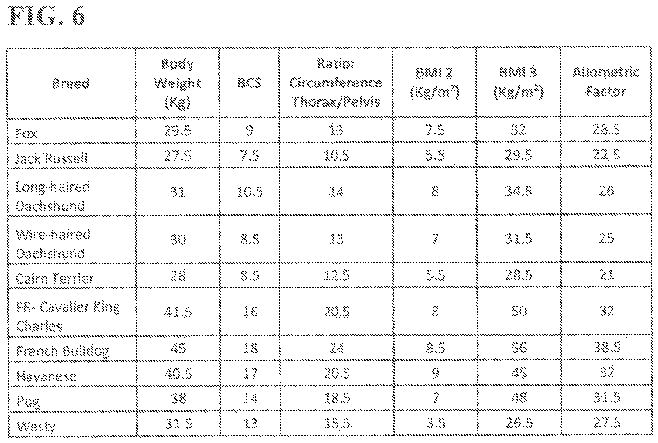

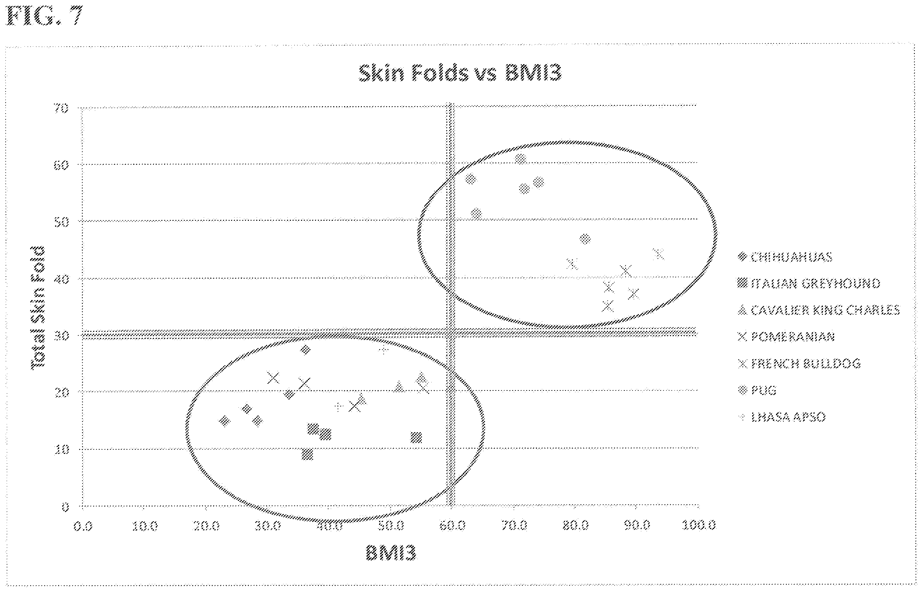

The data is shown in FIGS. 2-7. Averages and correlations were performed using Microsoft Excel. Principal Component Analysis was performed on dogs using BCS, total skinfold, thoracic/pelvic ration and BMI 3 variables with FactoMineR, a statistical package program of R (GNU General Public License).

PCA (Principal Component Analysis), a method to represent in the same plane different individuals according to several variables, was performed on the data. This allows a simplified way of viewing groups of individuals and relationships that can exist between them and between the variables used to generate the PCA.

Individuals (breeds) are represented in pink. Each black arrow represents an active variable (BCS, BMI 3, Total Skinfold, Thoracic/Pelvic ratio). The comparison of the sense and direction of two arrows indicates the relationship between two active variables; two arrows with the same sense and direction means that the variables are positively correlated (e.g., BMI 3 and Total Skinfold). Two arrows with the same direction but not with the same sense means that the variables are negatively correlated. Two arrows having perpendicular directions are not correlated (e.g. Total Skinfold and Thoracic/Pelvic ratio).

When an individual is in the sense and direction of an arrow, the individual has a high value on the variable, all the more so that the individual is located far from the origin of the arrow. On the graph, French Bulldog and Pug have a strong BMI 3 and important length of skinfold.

When an individual is in the opposite direction of an arrow, he individual has a low value on the variable, all the more so that the individual is located far from the origin of the arrow. On the graph, Italian Greyhound has a low BCS.

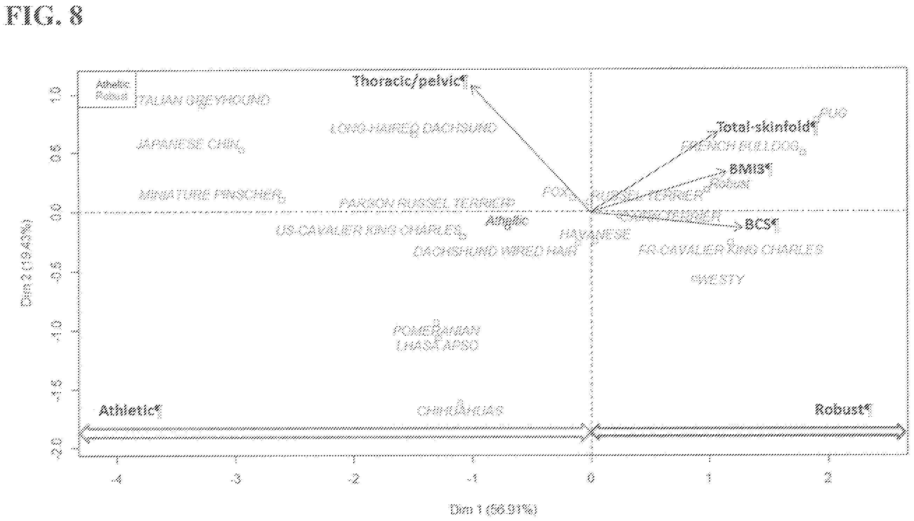

As shown in **FIG. 8****,** the PCA used the variables BMI 3, BCS, Total Skinfold, and Thoracic/Pelvic ratio. The graph demonstrates a strong correlation between BCS, BMI 3 and Total Skinfold, but that these variables are poorly correlated with Thoracic/Pelvic ratio.

It was striking to observe that the PCA separates the breeds according to their morphology quite effectively with the robust breeds on the right side and athletic breeds on the left side of the graph. Also shown is that some breeds clearly emerge as robust or athletic (French Bulldog, Italian Greyhound) while other breeds are more complicated to classify (Havanese, Fox). This tool can be used to classify new breeds after morphometric measurements are collected.

## Claims

1. A method of formulating a dog food composition for a small dog having a height, a sternum and a body length, the method comprising:
generating, while the small dog is erect on a floor, measurements of the height, the height of the sternum to the floor, and the body length;
using the measurements to determine the modified body mass index of the small dog, wherein said modified body mass index is body weight / [(height - height of the sternum to the floor) x body length];
categorizing the small dog as one of a plurality of body conformation types, the categorizing being based at least partially on the modified body mass index of the small dog; and
formulating a dog food composition for the small dog, wherein the dog food composition is formulated to contain feed ingredients and amounts thereof based on the categorizing of the small dog.

2. The method of claim 1, wherein the categorizing of the small dog categorizes the small dog as a robust body conformation type or an athletic body conformation type, the modified body mass index of the robust body conformation type is greater than 60 kg/m², and the modified body mass index of the athletic body conformation type is not greater than 60 kg/m².

3. The method of claim 2 wherein the ratio of energy from protein to energy from fat is greater for the dog food composition formulated for the small dog categorized as the robust body conformation type relative to the dog food composition formulated for the small dog categorized as the athletic body conformation type.

4. The method of claim 2 wherein the fat content is greater for the dog food composition formulated for the small dog categorized as the athletic body conformation type relative to the dog food composition formulated for the small dog categorized as the robust body conformation type.

5. The method of claim 1, wherein the categorizing is partially based on an energy requirement of the small dog.

6. The method of claim 5, wherein the categorizing of the small dog categorizes the small dog as a robust body conformation type or an athletic body conformation type;
for the robust body conformation type, the modified body mass index is greater than 60 kg/m² and/or the energy requirement is less than 120x(kilograms of ideal body weight)^{0.15} kilocalories per day; and
for the athletic body conformation type, the modified body mass index is not greater than 60 kg/m² and/or the energy requirement is more than 120x(kilograms of ideal body weight)^{0.15} kilocalories per day.

7. The method of claim 1, wherein the categorizing is partially based on a skin fold measurement of the small dog, the skin fold measurement is one or more measurements of the thickness of the subcutaneous fat layer of the small dog, and each of the one or more measurements is obtained in a different location on the small dog relative to the other measurements.

8. The method of claim 1, wherein the small dog has a weight less than 10 kg.

9. A method of selecting a food composition for a small dog having a height, a sternum and a body length, the method comprising:
measuring, while the small dog is erect on a floor, the height, the height of the sternum to the floor, and the body length;
determining a modified body mass index of the small dog, wherein the modified body mass index is body weight / [(height - height of the sternum to the floor) x body length];
categorizing the small dog as one of a plurality of body conformation types using the modified body mass index; and
selecting one of a plurality of food compositions based on the categorizing, wherein each of the plurality of food compositions respectively corresponds to one of the plurality of body conformation types.

10. The method of Claim 9, wherein a first food composition of the plurality of food compositions is formulated for a robust body conformation type of the small dog, the modified body mass index of the robust body conformation type is greater than 60 kg/m², a second food composition of the plurality of food compositions is formulated for an athletic body conformation type of the small dog, and the modified body mass index of the athletic body conformation type is not greater than 60 kg/ m².

11. The method of Claim 10, wherein the first food composition comprises a functional ingredient which limits fat accumulation selected from the group consisting of conjugated linoleic acid (CLA), isoflavones, and combinations thereof, and the second food composition does not contain the functional ingredient which limits fat accumulation.

12. The method of Claim 10, wherein a ratio of energy from protein to energy from fat and the fat content is different for the first food composition relative to the second food composition.

13. The method of claim 9, wherein the categorizing is partially based on a skin fold measurement of the small dog, the skin fold measurement is one or more measurements of the thickness of the subcutaneous fat layer of the small dog, and each of the one or more measurements is obtained in a different location on the small dog relative to the other measurements.

## Patentansprüche

1. Verfahren zum Formulieren einer Hundefutterzusammensetzung für einen kleinen Hund mit einer Höhe, einem Sternum und einer Körperlänge, wobei das Verfahren umfasst:
Erzeugen von Messungen der Höhe, der Höhe von Sternum zum Boden und der Körperlänge, während der kleine Hund aufrecht auf einem Boden steht;
Verwenden der Messungen, um den modifizierten Body-Mass-Index des kleinen Hundes zu bestimmen, wobei der modifizierte Body-Mass-Index Körpergewicht / [(Höhe - Höhe von Sternum zum Boden) x Körperlänge] ist;
Kategorisieren des kleinen Hundes als einen von mehreren Körperkonformationstypen, wobei die Kategorisierung mindestens teilweise auf dem modifizierten Body-Mass-Index des kleinen Hundes basiert; und
Formulieren einer Hundefutterzusammensetzung für den kleinen Hund, wobei die Hundefutterzusammensetzung so formuliert ist, dass sie Futterbestandteile und Mengen davon auf Basis der Kategorisierung des kleinen Hundes enthält.

2. Verfahren nach Anspruch 1, wobei das Kategorisieren des kleinen Hundes den kleinen Hund als einen robusten Körperkonformationstyp oder einen athletischen Körperkonformationstyp kategorisiert, wobei der modifizierte Body-Mass-Index des robusten Körperkonformationstyps größer als 60 kg/m² ist und der modifizierte Body-Mass-Index des athletischen Körperkonformationstyps nicht größer als 60 kg/m² ist.

3. Verfahren nach Anspruch 2, wobei das Verhältnis von Energie aus Protein zu Energie aus Fett für die Hundefutterzusammensetzung ist, die für den kleinen Hund formuliert ist, der als der robuste Körperkonformationstyp kategorisiert ist, größer ist als für die Hundefutterzusammensetzung, die für den kleinen Hund formuliert ist, der als der athletische Körperkonformationstyp kategorisiert ist.

4. Verfahren nach Anspruch 2, wobei der Fettgehalt für die Hundefutterzusammensetzung ist, die für den kleinen Hund formuliert ist, der als der athletische Körperkonformationstyp kategorisiert ist, größer ist als für die Hundefutterzusammensetzung, die für den kleinen Hund formuliert ist, der als der robuste Körperkonformationstyp kategorisiert ist.

5. Verfahren nach Anspruch 1, wobei das Kategorisieren teilweise auf einem Energiebedarf des kleinen Hundes basiert.

6. Verfahren nach Anspruch 5, wobei das Kategorisieren des kleinen Hundes den kleinen Hund als einen robusten Körperkonformationstyp oder einen athletischen Körperkonformationstyp kategorisiert;
für den robusten Körperkonformationstyp ist der modifizierte Body-Mass-Index größer als 60 kg/m² und/oder beträgt der Energiebedarf weniger als 120 x (Kilogramm ideales Körpergewicht)^{0,75} Kilokalorien pro Tag; und
für den athletischen Körperkonformationstyp ist der modifizierte Body-Mass-Index nicht größer als 60 kg/m² und/oder beträgt der Energiebedarf mehr als 120 x (Kilogramm ideales Körpergewicht)^{0,75} Kilokalorien pro Tag.

7. Verfahren nach Anspruch 1, wobei das Kategorisieren teilweise auf einer Hautfaltenmessung des kleinen Hundes basiert, wobei die Hautfaltenmessung eine oder mehrere Messungen der Dicke der subkutanen Fettschicht des kleinen Hundes ist und jede der einen oder mehreren Messungen an einer anderen Stelle an dem kleinen Hund in Bezug zu den anderen Messungen erhalten wird.

8. Verfahren nach Anspruch 1, wobei der kleine Hund ein Gewicht von weniger als 10 kg aufweist.

9. Verfahren zum Auswählen einer Futterzusammensetzung für einen kleinen Hund mit einer Höhe, einem Sternum und einer
Körperlänge, wobei das Verfahren umfasst:
Messen der Höhe, der Höhe von Sternum zum Boden und der Körperlänge, während der kleine Hund aufrecht auf einem Boden steht;
Bestimmen eines modifizierten Body-Mass-Index des kleinen Hundes, wobei der modifizierte Body-Mass-Index Körpergewicht / [(Höhe - Höhe von Sternum zum Boden) x Körperlänge] ist;
Kategorisieren des kleinen Hundes als einen aus einer Vielzahl von Körperkonformationstypen unter Verwendung des modifizierten Body-Mass-Index; und
Auswählen einer aus einer Vielzahl von Futterzusammensetzungen auf Basis der Kategorisierung, wobei jede aus der Vielzahl von Futterzusammensetzungen jeweils einem aus der Vielzahl von Körperkonformationstypen entspricht.

10. Verfahren nach Anspruch 9, wobei eine erste Futterzusammensetzung aus der Vielzahl von Futterzusammensetzungen für einen robusten Körperkonformationstyp des kleinen Hundes formuliert ist, der modifizierte Body-Mass-Index des robusten Körperkonformationstyps größer als 60 kg/m² ist, eine zweite Futterzusammensetzung aus der Vielzahl von Futterzusammensetzungen für einen athletischen Körperkonformationstyp des kleinen Hundes formuliert ist und der modifizierte Body-Mass-Index des athletischen Körperkonformationstyps nicht größer als 60 kg/m² ist.

11. Verfahren nach Anspruch 10, wobei die erste Futterzusammensetzung einen funktionellen Inhaltsstoff umfasst, der die Fettansammlung begrenzt, ausgewählt aus der Gruppe bestehend aus konjugierter Linolsäure (CLA), Isoflavonen und Kombinationen davon, und die zweite Futterzusammensetzung den funktionellen Inhaltsstoff, der die Fettansammlung begrenzt, nicht enthält.

12. Verfahren nach Anspruch 10, wobei ein Verhältnis von Energie aus Protein zu Energie aus Fett sowie der Fettgehalt bei der ersten Futterzusammensetzung anders ist als bei der zweiten Futterzusammensetzung.

13. Verfahren nach Anspruch 9, wobei das Kategorisieren teilweise auf einer Hautfaltenmessung des kleinen Hundes basiert, wobei die Hautfaltenmessung eine oder mehrere Messungen der Dicke der subkutanen Fettschicht des kleinen Hundes ist und jede der einen oder mehreren Messungen an einer anderen Stelle auf dem kleinen Hund relativ zu den anderen Messungen erhalten wird.

## Revendications

1. Procédé de formulation d'une composition alimentaire pour chiens destinée à un petit chien ayant une hauteur, un sternum et une longueur de corps, le procédé comprenant :
la génération, alors que le petit chien est debout sur un sol, de mesures de la hauteur, de la hauteur du sternum au sol, et de la longueur de corps ;
l'utilisation des mesures pour déterminer l'indice de masse corporelle modifié du petit chien, dans lequel ledit indice de masse corporelle modifié est poids corporel / [(hauteur - hauteur du sternum au sol) x longueur de corps] ;
la catégorisation du petit chien en tant qu'un parmi une pluralité de types de conformation corporelle, la catégorisation étant basée au moins partiellement sur l'indice de masse corporelle modifié du petit chien ; et
la formulation d'une composition alimentaire pour chiens destinée au petit chien, dans lequel la composition alimentaire pour chiens est formulée pour contenir des ingrédients alimentaires et des quantités de ceux-ci sur la base de la catégorisation du petit chien.

2. Procédé selon la revendication 1, dans lequel la catégorisation du petit chien catégorise le petit chien en tant que type de conformation corporelle robuste ou type de conformation corporelle athlétique, l'indice de masse corporelle modifié du type de conformation corporelle robuste est supérieur à 60 kg/m², et l'indice de masse corporelle modifié du type de conformation corporelle athlétique n'est pas supérieur à 60 kg/m².

3. Procédé selon la revendication 2 dans lequel le rapport de l'énergie provenant de protéines à l'énergie provenant de matière grasse est plus grand pour la composition alimentaire pour chiens formulée pour le petit chien catégorisé en tant que type de conformation corporelle robuste par rapport à la composition alimentaire pour chiens formulée pour le petit chien catégorisé en tant que type de conformation corporelle athlétique.

4. Procédé selon la revendication 2 dans lequel la teneur en matières grasses est plus grande pour la composition alimentaire pour chiens formulée pour le petit chien catégorisé en tant que type de conformation corporelle athlétique par rapport à la composition alimentaire pour chiens formulée pour le petit chien catégorisé en tant que type de conformation corporelle robuste.

5. Procédé selon la revendication 1, dans lequel la catégorisation est partiellement basée sur un besoin énergétique du petit chien.

6. Procédé selon la revendication 5, dans lequel la catégorisation du petit chien catégorise le petit chien en tant que type de conformation corporelle robuste ou type de conformation corporelle athlétique ;
pour le type de conformation corporelle robuste, l'indice de masse corporelle modifié est supérieur à 60 kg/m² et/ou le besoin énergétique est inférieur à 120x(kilogrammes de poids corporel idéal)^{0,75} kilocalories par jour ; et
pour le type de conformation corporelle athlétique, l'indice de masse corporelle modifié n'est pas supérieur à 60 kg/m² et/ou le besoin énergétique est supérieur à 120x(kilogrammes de poids corporel idéal)^{0,75} kilocalories par jour.

7. Procédé selon la revendication 1, dans lequel la catégorisation est partiellement basée sur une mesure de pli cutané du petit chien, la mesure de pli cutané est une ou plusieurs mesures de l'épaisseur de la couche grasse sous-cutanée du petit chien, et chacune parmi la ou les mesures est obtenue dans un emplacement différent sur le petit chien par rapport aux autres mesures.

8. Procédé selon la revendication 1, dans lequel le petit chien a un poids inférieur à 10 kg.

9. Procédé de sélection d'une composition alimentaire pour un petit chien ayant une hauteur, un sternum et une longueur de corps, le procédé comprenant :
la mesure, alors que le petit chien est debout sur un sol, de la hauteur, de la hauteur du sternum au sol, et de la longueur de corps ;
la détermination d'un indice de masse corporelle modifié du petit chien, dans lequel l'indice de masse corporelle modifié est poids corporel / [(hauteur - hauteur du sternum au sol) x longueur de corps] ;
la catégorisation du petit chien en tant qu'un parmi une pluralité de types de conformation corporelle en utilisant l'indice de masse corporelle modifié ; et
la sélection d'une parmi une pluralité de compositions alimentaires sur la base de la catégorisation, dans lequel chacune parmi la pluralité de compositions alimentaires correspond respectivement à l'un parmi la pluralité de types de conformation corporelle.

10. Procédé selon la revendication 9, dans lequel une première composition alimentaire de la pluralité de compositions alimentaires est formulée pour un type de conformation corporelle robuste du petit chien, l'indice de masse corporelle modifié du type de conformation corporelle robuste est supérieur à 60 kg/m², une deuxième composition alimentaire de la pluralité de compositions alimentaires est formulée pour un type de conformation corporelle athlétique du petit chien, et l'indice de masse corporelle modifié du type de conformation corporelle athlétique n'est pas supérieur à 60 kg/m².

11. Procédé selon la revendication 10, dans lequel la première composition alimentaire comprend un ingrédient fonctionnel qui limite une accumulation de graisses choisi dans le groupe constitué d'acide linoléique conjugué (CLA), isoflavones, et des combinaisons de ceux-ci, et la deuxième composition alimentaire ne contient pas l'ingrédient fonctionnel qui limite une accumulation de graisses.

12. Procédé selon la revendication 10, dans lequel un rapport de l'énergie provenant de protéines à l'énergie provenant de matière grasse et la teneur en matières grasses est différent pour la première composition alimentaire par rapport à la deuxième composition alimentaire.

13. Procédé selon la revendication 9, dans lequel la catégorisation est partiellement basée sur une mesure de pli cutané du petit chien, la mesure de pli cutané est une ou plusieurs mesures de l'épaisseur de la couche grasse sous-cutanée du petit chien, et chacune parmi la ou les mesures est obtenue dans un emplacement différent sur le petit chien par rapport aux autres mesures.
